# EUROPÄISCHE PATENTANMELDUNG

(11) **EP 0 657 143 A1**
(43) Veröffentlichungstag der Anmeldung: **14.06.1995**
(21) Anmeldenummer: 94118888.0
(22) Anmeldetag: 30.11.1994
(51) Int. Cl.: A61C 5/06, A61M 5/31

(54) **Vorrats- und Dosierspritze für zähflüssige Dentalmassen**

(30) Priorität: 10.12.1993 DE 9319007 U
(71) Anmelder: Mühlbauer, Ernst, D-22547 Hamburg (DE)
(72) Erfinder: Mühlbauer, Ernst, D-22547 Hamburg (DE)
(74) Vertreter: Glawe, Delfs, Moll & Partner

(57) **Zusammenfassung**

Vorrats- und Dosierspritze für zähflüssige Dentalmassen mit einem einen Kolben enthaltenden Spritzenkörper, der in einer Ausbringöffnung mündet. Die Ausbringöffnung schließt sich verengungsfrei an den Spritzenkörper an. Dadurch wird eine Separation der Massenkomponenten im Oberflächenbereich des austretenden Strangs verhütet. Vorzugsweise erweitert sich der Durchmesser des Spritzenkörpers zur Ausbringöffnung hin.

## Beschreibung

Die Erfindung betrifft eine Vorrats- und Dosierspritze für eine zähflüssige Dentalmasse mit einem Spritzenkörper, der eine einen Kolben enthaltende Bohrung aufweist, die in einer Ausbringöffnung mündet.

Unter zähflüssigen Dentalmassen sind vornehmlich stark gefüllte Harze zu verstehen, die für Füllungen sowie für Verblendungen an natürlichen Zähnen und Zahnprothesen bestimmt sind. Dieser gesamte Anwendungsbereich ist gemeint, wenn im folgenden einfachheitshalber nur von Füllungen gesprochen wird. Die Dentalmassen bestehen aus einem hohen Füllstoffanteil und einer dünn- bis honigflüssigen Harzphase, die ihrerseits aus mehreren Komponenten unterschiedlicher Zähigkeit bestehen kann. Die Dentalmassen sind zwar äußerst zäh, aufgrund des flüssigen Harzanteils aber jedenfalls fließfähig. Dies unterscheidet sie von Amalgam, das auch im knetbaren Zustand niemals flüssig ist.

Zum Bevorraten, Vorlegen und Dosieren dieser Massen werden Spritzen verwendet, deren Spritzenkörper die Masse in einer Zylinderbohrung enthält. Mittels eines Kolbens kann die Masse durch eine Ausbringöffnung ausgepreßt werden. Diese Ausbringöffnung hat in der Regel einen Durchmesser von einigen Millimetern. Der Spritzenkörper und der Kolben haben einen größeren Durchmesser, damit das erforderliche Volumen in mäßiger Länge des Spritzenkörpers untergebracht werden kann. Je größer der Durchmesser im Vorratsteil der Applikationsspritze ist, um so mehr Dentalmasse kann darin bevorratet sein; je kleiner die Ausbringöffnung ist, um so zielgenauer kann die Masse appliziert werden. Bei den bekannten Spritzen ist also immer eine Verengung zwischen der Bohrung des Spritzenkörpers und der Ausbringöffnung vorhanden. Zwar ist es bekannt, daß es im Hinblick auf die Ausbringkräfte zweckmäßig sein kann, das Flächenverhältnis zwischen Spritzenkörper und Ausbringöffnung gering zu halten (EP-B-0220551), jedoch wurde niemals auf die Verengung verzichtet. Dies ist wegen der Fließfähigkeit der Masse auch nicht erforderlich.

Der Erfinder hat erkannt, daß beim Ausbringen zäher Füllmassen aus den bekannten Vorratsspritzen an der Oberfläche des ausgepreßten Strangs gewisse Veränderungen stattfinden, die sich nachteilig auswirken können. Er hat beobachtet, daß die Oberfläche nach dem Austritt des Strangs zunächst rauh und rissig ist, und bei näherer Untersuchung herausgefunden, daß dies zusammenhängt mit Separationserscheinungen zwischen den in der Masse enthaltenen Phasen unterschiedlicher Konsistenz. Zum einen ergeben die Separationserscheinungen bereichsweise Abweichungen von dem gewollten Zusammensetzungsverhältnis. Zum anderen können sich durch die Rauhigkeit und Rissigkeit der Oberfläche des austretenden Strangs Lufteinschlüsse ergeben, die in der Masse und der daraus hergestellten Zahnfüllung verbleiben und zu Mängeln führen. Er hat ferner herausgefunden, daß diese Erscheinungen von den besonderen Strömungsverhältnissen herrühren, denen die Masse beim Ausbringen aus der Spritze unter gleichzeitiger Einschnürung ihres Querschnitts ausgesetzt ist.

Die Erfindung besteht daher in der Lehre, eine Qualitätsverschlechterung des aus der Spritze austretenden Strangs dadurch zu vermeiden, daß die Bohrung der Spritze bis hin zu ihrer Ausbringöffnung verengungsfrei ausgeführt ist.

Verengungsfreie Applikationsröhrchen sind für Amalgam bekannt (WO-93/16653, FR-A-1191538, US-A-1694524). Der Zahnarzt mischt das Amalgam separat, überführt Bröckchen des gemischten Amalgams in das Applikationsröhrchen und appliziert es damit in der zu füllenden Zahnkavität. Es ist offensichtlich, daß derartige Amalgam-Applikationsröhrchen aus drei Gründen verengungsfrei sein müssen. Zum einen ist dies erforderlich, weil die zu applizierenden Amalgambröckchen durch die Ausbringöffnung hindurch in das Applikationsröhrchen eingefüllt werden. Zum anderen verbietet die nicht fließfähige Konsistenz des Amalgams jegliche Verengung. Es bestand kein Anlaß, die verengungsfreie Form der für Amalgam bestimmten Applikationsröhrchen auf die für zähflüssige Harzmassen bestimmten Applikationsspritzen zu übertragen, weil dies nach der Kenntnis des Fachmanns aus den weiter oben angegebenen Gründen nachteilig gewesen wäre. Er konnte nicht wissen, daß die Verengungsfreiheit bei fließfähiger Dentalmasse im Gegensatz zu Amalgam zu einer Qualitätsverbesserung des aus der Applikationsspritze austretenden Materials führt. Die Erfindung konnte durch die bekannten Amalgam-Applikationsröhrchen nicht nahegelegt werden, da bei diesen die Verengungsfreiheit auf anderen Gesichtspunkten beruht und ihnen nicht die Lehre entnommen werden kann, daß Verengungfreiheit bei fließfähigen Harzmassen zu einer Qualitätsverbesserung führt.

Eine weitere Verbesserung im Sinne der Erfindung läßt sich dadurch erreichen, daß der lichte Durchmesser der Spritzenbohrung sich zur Ausbringöffnung hin ein wenig erweitert. Dadurch wird die Reibung des bewegten Strangs an der Spritzenwandung verringert oder teilweise aufgehoben mit dem Ergebnis, daß die laminaren Geschwindigkeitsunterschiede und damit die Scherspannungen im bewegten Strang und die daraus resultierende Neigung zu entmischender Bewegung ihrer Komponenten gänzlich vermieden werden.

Da die Verengungsfreiheit und ggf. Erweiterung des Spritzenkörpers verbunden ist mit einer Verringerung der Ausbringkräfte, genügt oftmals die Handkraft zum Vorschieben des Kolbens. Jedoch kann es vorteilhaft sein, die Kolbenkraft mittels einer Untersetzung, beispielsweise einer Gewindespindel, zu vergrößern. Im Hinblick auf die wünschenswerte Größe der Ausbringöffnung ist der Durchmesser der Spritzenbohrung und des Kolbens zweckmäßigerweise geringer als bei bekannten Vorratsspritzen, so daß auch aus diesem Grund die Ausbringkraft reduziert ist. Der genannte Durchmesser liegt zweckmäßigerweise in der Größenordnung von 3 bis 6 mm, vorzugsweise 4 bis 5 mm.

Die erfindungsgemäße Spritze nimmt vorzugsweise ein Füllvolumen auf, das für viele Anwendungsfälle ausreicht. Jedoch ist es auch möglich, das Füllvolumen auf einmaligen Bedarf zu beschränken. Sie wird dann zweckmäßigerweise so ausgeführt, daß sie mit einem geeigneten Applikator gekuppelt werden kann.

Die Erfindung wird im folgenden näher unter Bezugnahme auf die Zeichnung erläutert, die vorteilhafte Ausführungsbeispiele veranschaulicht. Es zeigen:
- Fig. 1: einen Längsschnitt durch eine Vorratsspritze mit Handvorschub des Kolbens,
- Fig. 2: eine mit Gewindespindel ausgerüstete Ausführungsform der Spritze gemäß Fig. 1,
- Fig. 3: einen Applikator mit Spritzenkapsel und
- Fig. 4: die im Ausführungsbeispiel gemäß Fig. 3 verwendete Spritzenkapsel.

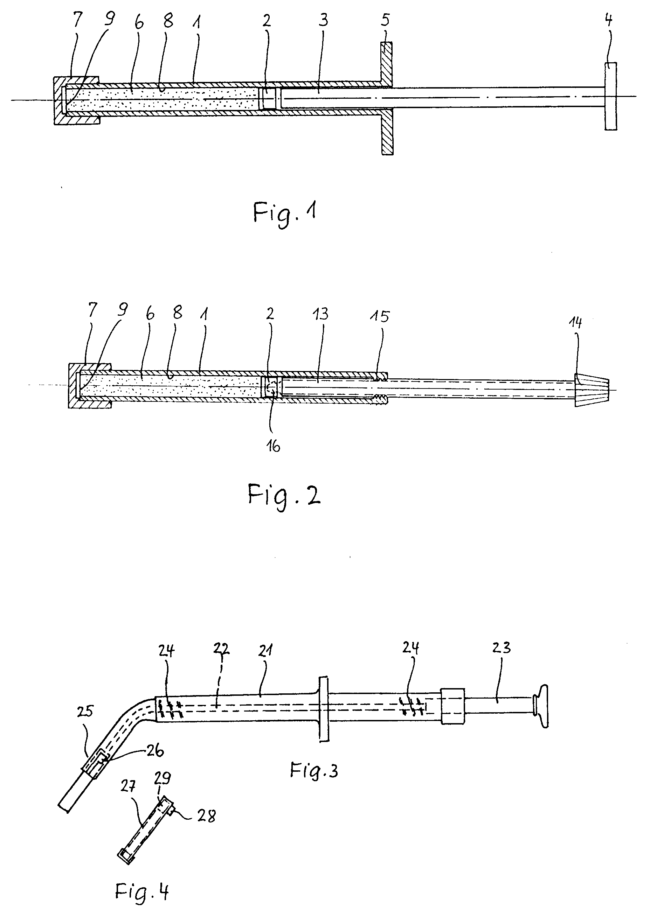

Bei der Ausführung gemäß Fig. 1 befindet sich in der Bohrung 8 des im wesentlichen zylindrischen Spritzenkörpers 1 der Kolben 2, der mittels einer Kolbenstange 3, die mit dem Kolben 2 einstückig verbunden sein kann aber nicht muß, vorgetrieben werden kann. Zu diesem Zweck befindet sich am Ende der Kolbenstange 3 eine Handhabe 4 für den Daumen und am Spritzenkörper 1 ein Widerlager 5 für Mittelfinger und Zeigefinger der die Spritze betätigenden Hand. Die Masse 6 befindet sich in der Bohrung 8 des Spritzenkörpers 1, die einen lichten Durchmesser in der Größenordnung von 5mm aufweist, der sich von hinten nach vorne zur Ausbringöffnung 9 hin stetig um wenige Zehntel Millimeter, beispielsweise um 0,1 bis 0,2 mm, vergrößert. Die Ausbringöffnung 9 kann durch eine Kappe 7 verschlossen werden. Die Länge des Spritzenkörpers liegt beispielsweise bei 7 bis 8 cm. Es kann daher eine Menge der Füllmasse aufgenommen werden, die für eine Vielzahl von Füllungen ausreicht. Die Masse läßt sich aus der Spritze leicht vorlegen und dosieren, da die Vortriebskraft für Handbetätigung hinreichend gering ist. Dank der Zähigkeit der Masse ist der Vorschub gleichmäßig.

Das Ausführungsbeispiel gemäß Fig. 2 unterscheidet sich von dem nach Fig. 1 dadurch, daß die Kolbenstange als Gewindespindel 13 ausgebildet ist, deren Gewinde mit einem Muttergewinde 15 am hinteren Ende des Spritzenkörpers 1 zusammenwirkt. Der Griff am Spritzenkörper für Handbetätigung fehlt. Am hinteren Ende der Spindel ist ein Betätigungsteil 14 vorgesehen, mittels dessen die Spindel gedreht werden kann. Die Spindel 13 ist ein von dem Kolben 2 gesonderter Teil. Sie besitzt an ihrem vorderen Ende ein Rastglied 16, das in eine hinterschnittene Bohrung im Kolben zugfest eingerastet werden kann, damit durch Drehung der Spindel in der zum Vortrieb entgegengesetzten Richtung der Kolben zurückgezogen werden kann. Dies ist vorteilhaft, damit ein Teil der teuren Masse, der möglicherweise über die Menge des augenblicklichen Bedarfs hinaus ausgebracht wurde, in die Spritze zurückgesaugt werden kann.

Wenn man das Füllvolumen der Spritze auf die Menge beschränkt, die für einen Bedarfsfall ausreicht, gelangt man zu sehr kleinen Spritzenkörpern, wie sie in Fig. 4 dargestellt sind und die hier als Spritzenkapseln bezeichnet werden. Da es sehr aufwendig wäre, eine solche Spritzenkapsel mit Betätigungseinrichtungen auszurüsten, werden Sie zweckmäßigerweise in Verbindung mit einem sogenannten Applikator benutzt, wie er in Fig. 3 dargestellt ist. Dieser weist einen langgestreckten, hohlzylindrischen Körper 21 auf, in welchem eine flexible Stange 22 mittels einer Handhabe 23 gegenüber die Kraft einer Feder vorschiebbar ist, wie bei 24 angedeutet ist. Am vorderen Ende, das zweckmäßigerweise in der dargestellten Form gebogen ist, bildet der Applikator ein Mundstück 25, das mit einem Bajonettausschnitt 26 versehen ist. Das rohrförmige Mundstück 25 hat einen Innendurchmesser, der wenig größer ist als der Außendurchmesser der Spritzenkapsel 27, die an ihrem hinteren Ende einen seitlichen Bajonettvorsprung 28 aufweist, der in die Bajonettausnehmung 26 des Mundstücks 25 paßt. Das vordere Ende der flexiblen Stange 22 liegt im Ausgangszustand im Mundstück 25 mittig hinter der Spritzenkapsel 27. Wird sie durch Druck auf die Betätigungseinrichtung 23 vorgeschoben, so preßt sie den Kolben 29 der Spritzenkapsel vor, wodurch diese entleert wird. Die Bohrung der Spritzenkapsel 27 ist ebenso zylindrisch oder erweitert sich zu ihrer Mündungsöffnung hin, wie dies weiter oben beschrieben wurde.

Was unter einer zähflüssigen Masse und deren Zähigkeit bzw. Fließfähigkeit zu verstehen ist, wird bei folgendem Beispiel deutlich. Ein im Querschnitt runder Strang von 5mm Durchmesser und 10mm Länge wird auf einer horizontalen Fläche ausgebracht. Nach einer halben Stunde hat der Strang noch immer etwa kreisförmigen Querschnitt. Seine auf der tragenden Fläche aufliegende Unterseite hat sich aber auf einer Breite von etwa 2 mm abgeplattet.

## Patentansprüche

1. Vorrats- und Dosierspritze für eine zähflüssige Dentalmasse mit einem Spritzenkörper (1), der eine einen Kolben und die Dentalmasse enthaltende Bohrung (8) aufweist, die in einer Ausbringöffnung (9) mündet, dadurch gekennzeichnet, daß die Ausbringöffnung (9) sich verengungsfrei an die Bohrung (8) anschließt.

2. Spritze nach Anspruch 1, dadurch gekennzeichnet, daß die Bohrung (8) sich zur Ausbringöffnung (9) hin schwach erweitert.

3. Spritze nach Anspruch 1 oder 2, dadurch gekennzeichnet, daß der Kolben (2) bis zur Ausbringöffnung (9) vorschiebbar ist.

4. Spritze nach einem der Ansprüche 1 bis 3, dadurch gekennzeichnet, daß der Kolben übersetzungsfrei von Hand vorschiebbar ist.

5. Spritze nach einem der Ansprüche 1 bis 3, dadurch gekennzeichnet, daß der Kolben (2) mit einer Kolbenstange (3) verbunden ist, die über ein Gewinde mit dem Spritzenkörper (1) verbunden ist.

6. Spritze nach einem der Ansprüche 1 bis 5, dadurch gekennzeichnet, daß der Kolben (2) mit einer Kolbenstange (3) zugbelastbar verbunden ist.

7. Spritze nach einem der Ansprüche 1 bis 4, dadurch gekennzeichnet, daß sie mit einem Füllvolumen für einmaligen Bedarf ausgestattet ist und mit einer Kupplungseinrichtung zum Verbinden mit einem Applikator versehen ist.
